# EUROPEAN PATENT APPLICATION

(11) **EP 4 134 079 A1**
(43) Date of publication of application: **15.02.2023**
(21) Application number: 21785494.2
(22) Date of filing: 23.03.2021
(51) Int. Cl.: A61K 31/4439, A61P 13/12

(54) **AGENT FOR TREATING CONTRAST-INDUCED ACUTE KIDNEY INJURY**

(30) Priority: 08.04.2020 KR 20200042899; 22.03.2021 KR 20210036838
(71) Applicant: Aptabio Therapeutics Inc., Yongin-si, Gyeonggi-do 16954 (KR)
(72) Inventor: LEE, Soo Jin, Suwon-si, Gyeonggi-do 16709 (KR); MOON, Sung Hwan, Suwon-si, Gyeonggi-do 16222 (KR)
(74) Representative: dompatent von Kreisler Selting Werner - Partnerschaft von Patent- und Rechtsanwälten mbB
(86) International application number: PCT/KR2021/003536
(87) International publication number: WO 2021/206316

(57) **Abstract**

The present invention provides a pharmaceutical composition for protecting the kidney by reducing contrast media-induced nephrotoxicity or for treating contrast media-induced acute kidney injury, comprising a compound of Formula 1, which is a pyrazole derivative, or a pharmaceutically acceptable salt thereof.

## Description

### [Technical Field]

This application claims the benefit of Korean Patent Application No. 10-2020-0042899 filed on April 8, 2020, and Korean Patent Application No. 10-2021-0036838 filed on March 22, 2021, with the Korean Intellectual Property Office, the disclosure of which are herein incorporated by reference in their entirety.

The present invention relates to a pyrazole derivative useful for protecting the kidney by reducing contrast media-induced nephrotoxicity or for preventing and treating contrast media-induced acute kidney injury, a method for preparing the same, and a pharmaceutical composition thereof.

### [Background Art]

Contrast media (CM) is a drug that is introduced into the stomach, intestinal tract, blood vessels, cerebrospinal cavity, joint cavity, and the like to increase the contrast of images by artificially increasing the difference in the absorption of X-rays in each tissue so that the tissue or blood vessels can be seen clearly during radiological examinations such as magnetic resonance imaging (MRI) or computed tomography (CT) imaging. By using a contrast media, it improves the diagnostic value by making it possible to distinguish a biological structure or lesion well from its surroundings.

Contrast media is generally divided into negative and positive contrast media, wherein the negative contrast media transmits more X-rays than surrounding tissues to display images. Positive contrast media include iodine-containing contrast media, barium sulfate, and the like, and negative contrast media include air, gas, carbon dioxide, and the like.

Some people are sensitive to contrast media and may cause an allergic reaction, resulting in rash, itching, fever, nausea, vomiting, joint pain, hemorrhagic predisposition, and the like.

As side effects of contrast media, shock or anaphylactic reaction may occur rarely, and hypersensitivity reactions such as hives, flushing, rash, and itching may occur, and seriously, acute kidney injury may occur.

In particular, although the exact mechanism of contrast media-induced acute kidney injury is not known, it occurs after administration of iodine-based contrast media and is known to be the major cause of hospital-acquired acute kidney injury.

Acute kidney injury due to contrast media-induced acute kidney injury is defined as an increase in the level of creatinine in the blood by 25% or more or 0.5 mg/dl or more compared to the existing level within 48 hours of use, and this case, the patient should not have other causes that cause kidney injury, i.e., a drop in blood pressure, other nephrotoxicity, and the like.

In general, the level of creatinine in the blood reaches its peaks on day 3-5 after administration of contrast media, and then returns to the previous level within 7-10 days. Contrast media-induced acute kidney injury accounts for about 12% of hospital-acquired acute kidney injury, and is one of the three major causes of acute kidney injury in hospitalized patients, along with ischemic acute kidney injury (42%) and acute kidney injury due to urinary tract obstruction (18%). It has been reported that the incidence of contrast media-induced acute kidney injury in patients with normal renal function is low (0-5%), but the incidence in patients with reduced renal function increases to 12-27%. In particular, it has been reported that the incidence in high-risk patients, such as patients with dehydration, diabetic nephropathy, kidney damage, volume depletion or congestive heart failure, and elderly patients increased to 50%, and it has been reported that dialysis is necessary in 15%.

Despite these risks, especially in high-risk and elderly patients with major comorbidities, the use of radiographic contrast media for computed tomography and vascular intervention is rapidly increasing to more than 20% in those in their 50s or older. Therefore, in a situation in which the elderly patient group and the patient group with diabetes, hypertension and heart failure vulnerable to contrast media-induced kidney injury are increasing among the subject patients undergoing examination using a contrast media, the development of prevention and treatment methods to reduce contrast media-induced nephrotoxicity is very urgent.

Although the pathophysiology of contrast media-induced kidney disease has not yet been clearly elucidated, it is assumed that ischemic injury caused by a decrease in renal blood flow and direct renal tubular cell injury caused by contrast media are the main mechanisms.

In the case of ischemic injury, it is known that due to changes in hormones that regulate the amount of renal blood flow after administration of contrast media, the vasoconstrictor hormones adenosine and endothelin increase and the vasodilator hormones nitric oxide (NO) and prostaglandin decrease, and injuries due to reduced blood flow and hypoxia, especially in the region of the renal medulla occur. Characteristically, it is known that the ischemic injury and renal tubular cell injury ultimately increase the synthesis of free oxygen radicals in the renal tissue, causing extensive oxidative stress, which in turn causes renal cell injury due to the increase in inflammation and apoptosis caused by increased cytokines.

In the case of contrast media-induced kidney disease, since the method to block the progression of kidney injury that has already occurred is not clear and the induction time is clear, as in the case of other acute kidney injury, efforts are mainly made to block its occurrence through active prevention, and several studies are being conducted to reduce kidney injury caused by contrast media.

It is known that the general treatment method to prevent contrast media-induced nephrotoxicity is to try to prevent electrolyte imbalance while implementing sufficient fluid supply, but there is no definitive treatment or prevention method to prevent the progression of kidney injury, and in some cases, dialysis is necessary, and thus, more research is needed to develop a preventive method to prevent it from occurring at an early stage.

Recently, at the laboratory level, it has been reported that endopeptidase inhibitors or some aromatic-cationic peptides may prevent acute kidney injury caused by contrast media.

However, most studies are observational studies with laboratory or small-scale patients, and so far, the conclusion on whether contrast media-induced kidney injury may be effectively prevented through the use of these drugs is unclear, and there is no specific treatment method for contrast media-induced kidney injury.

Therefore, the present inventors have completed the present invention by determining that it is still necessary to develop a preventive and therapeutic agent for acute kidney injury caused by contrast media in spite of various studies.

On the other hand, none of the prior art document discloses that the pyrazole-based compound of the present invention is effective in preventing and treating contrast media-induced acute kidney injury.

### [Prior Art Documents]

(Patent Document 1) Korean Patent No. 10-1280160
(Patent Document 2) Korean Patent No. 10-1886894
(Patent Document 3) U.S. Laid-Open Patent Publication No. US 2019-0388492
(Non-Patent Document 1) Persson PB, Hansell P, Liss P. Pathophysiology of contrast medium-induced nephropathy. Kidney Int 2005;68:14-22.
(Non-Patent Document 2) Heyman SN, Reichman J, Brezis M. Pathophysiology of radiocontrast nephropathy: a role for medullary hypoxia. Invest Radiol 1999;34:685-691.

### [Disclosure]

### [Technical Problem]

It is an object of the present invention to provide a pharmaceutical composition comprising a compound of Formula 1 or a pharmaceutically acceptable salt thereof.

It is another object of the present invention to provide a pharmaceutical composition for protecting a kidney by reducing contrast media-induced nephrotoxicity, comprising a compound of Formula 1 or a pharmaceutically acceptable salt thereof.

It is another object of the present invention to provide a pharmaceutical composition for treating and preventing contrast media-induced acute kidney injury, comprising a compound of Formula 1 or a pharmaceutically acceptable salt thereof.

It is another object of the present invention to provide a contrast media-induced kidney injury reduction effect, blood creatinine level improvement effect, and renal tubular injury improvement effect using a compound of Formula 1 or a pharmaceutically acceptable salt thereof.

It is another object of the present invention to provide a method of protecting a kidney by reducing contrast media-induced nephrotoxicity, or a method of preventing or treating contrast media-induced acute kidney injury, by administering a compound of Formula 1 or a pharmaceutically acceptable salt thereof to an individual.

It is another object of the present invention to provide the use of a compound of Formula 1 or a pharmaceutically acceptable salt thereof for preventing or treating contrast media-induced acute kidney injury.

### [Technical Solution]

In order to achieve the above objects, the present invention provides a composition for protecting a kidney by reducing contrast media-induced nephrotoxicity or a pharmaceutical composition for preventing and improving or treating contrast media-induced acute kidney injury, comprising a pyrazole-based compound represented by following Formula 1 or a pharmaceutically acceptable salt thereof as an active ingredient. 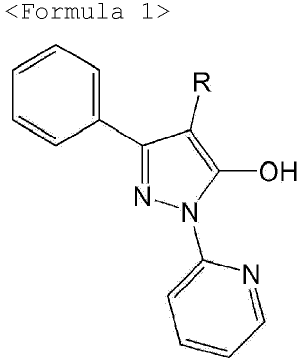 wherein R is a linear or branched alkyl group having 1 to 10 carbon atoms.

### [Advantageous Effects]

The pyrazole-based compound according to the present invention and a pharmaceutically acceptable salt thereof may effectively alleviate symptoms of acute kidney injury induced by administration of contrast media, and thus may be usefully used to protect a kidney by reducing contrast media-induced nephrotoxicity or to prevent or treat contrast media-induced acute kidney injury.

### [Description of Drawings]

Figure 1 shows results of analyzing the effect of the compound of the present invention in an animal model with contrast media-induced acute kidney injury.
Figure 2 shows a result of analyzing the effect of the compound of the present invention on improving the blood urea nitrogen (BUN) level in an animal model with contrast media-induced acute kidney injury.
Figure 3 shows a result of analyzing the effect of the compound of the present invention on improving the blood creatinine level in an animal model with contrast media-induced acute kidney injury.
Figure 4 shows results of analyzing the effect of the compound of the present invention on reducing the kidney injury markers NGAL, KIM-1 and microproteinuria (albumin) in an animal model with contrast media-induced acute kidney injury.
Figure 5 shows a result of analyzing the effect of the compound of the present invention on improving the renal tubular injury in an animal model with contrast media-induced acute kidney injury.
Figure 6 shows a result of analyzing the effect of the compound of the present invention on improving the inflammation of the kidney tissue in an animal model with contrast media-induced acute kidney injury.
Figure 7 shows a result of analyzing the effect of the compound of the present invention on reducing the infiltration of inflammatory cells within the kidney tissue in an animal model with contrast media-induced acute kidney injury.
Figure 8 shows results of analyzing the effect of the compound of the present invention on reducing activated oxygen that is an indicator of oxidative stress within the kidney tissue in an animal model with contrast media-induced acute kidney injury.
Figure 9 shows a result of analyzing the effect of the compound of the present invention on reducing nitrotyrosine that is a marker of oxidative stress within kidney tissue in an animal model with contrast media-induced acute kidney injury.

### [Best Mode]

Hereinafter, the present invention will be described in more detail with reference to embodiments.

However, the present invention is not limited by the embodiments that have been represented by way of example, and the present invention is defined only by the scope of the appended claims. In addition, even if it is a constitution essential for practicing the present invention, a specific description of the constitution that may be easily practiced by the skilled artisan will be omitted.

The terms and words as used in the present specification and claims should not be construed as limited to conventional or dictionary meanings, but should be construed as the meaning and concept consistent with the technical idea of the present invention based on the principle that the inventor can appropriately define the concept of the term to describe its own invention in the best way.

The terms used in the present invention are for the purpose of describing specific embodiment only and are not intended to limit the present invention. Singular expressions include plural expressions unless the context clearly indicates otherwise. In the present invention, terms such as "comprise" and "have" are intended to indicate that there is a feature, number, step, operation, component, part, or combination thereof described in the specification, and it should be understood that the terms do not exclude in advance the possibility of the presence or addition of one or more other features, numbers, steps, operations, components, parts, or combinations thereof.

As the number of diagnostic methods using contrast media increases, the use of the contrast media increases, and at the same time, side effects caused by the contrast media are also a problem. In addition to hypersensitivity reactions such as allergy due to contrast media, seriously, acute kidney injury may occur. In addition, it is known that an increasing number of elderly patients with major underlying diseases such as diabetes and hypertension and the increasing use of radiocontrast media for these patients are also one of the main causes of hospital-acquired acute kidney injury.

However, as a treatment method for contrast media-induced acute kidney injury, only a sufficient fluid supply and treatment to prevent electrolyte imbalance have been reported, and a method or established treatment for preventing contrast media-induced acute kidney injury have not been specifically reported.

Accordingly, the present invention provides a pharmaceutical composition capable of preventing or treating acute kidney injury caused by contrast media, comprising one or more compounds selected from the pyrazole-based compound represented by Formula 1 or a pharmaceutically acceptable salt thereof.

The pyrazole-based compound used in the present invention is represented by the following Formula 1: 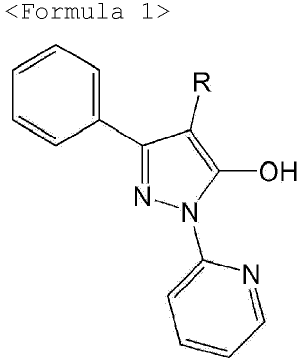 wherein R is a linear or branched alkyl group having 1 to 10 carbon atoms.

The pharmaceutically acceptable salt of the pyrazole-based compound included in the pharmaceutical composition of the present invention refers to salts that retain the biological effectiveness and properties of the parent compound and are not harmful biologically or otherwise when administered in a single dosage. In addition, it refers to a salt commonly used in the pharmaceutical industry.

Specifically, pharmaceutically acceptable base addition salts may be prepared from inorganic and organic bases. Salts derived from inorganic bases may include, but are not limited to, sodium, potassium, lithium, ammonium, calcium, and magnesium salts. Salts derived from organic bases include, but are not limited to, salts of primary, secondary, and tertiary amines; substituted amines including naturally occurring substituted amines; and isopropylamine, trimethylamine, diethylamine, triethylamine, tripropylamine, ethanolamine, 2-dimethylaminoethanol, tromethamine, lysine, arginine, histidine, caffeine, procaine, hydrabamine, choline, betaine, ethylenediamine, glucosamine, N-alkylglucamine, theobromine, purine, piperazine, piperidine, and/or cyclic amines including N-ethylpiperidine.

It should be also understood that other carboxylic acid derivatives, specifically carboxylic acid amides, including carboxamides, lower alkyl carboxamides, di(lower alkyl) carboxamides, and the like, are also useful in the practice of the present invention.

Additionally, pharmaceutically acceptable acid addition salts may be prepared from inorganic and organic acids. Salts derived from inorganic acids include hydrochloric acid, hydrobromic acid, sulfuric acid, nitric acid, phosphoric acid, perchloric acid, iodic acid, tartaric acid, and the like. Salts derived from organic acids may include, but are not limited to, acetic acid, trifluoroacetic acid, propionic acid, glycolic acid, gluconic acid, galacturonic acid, glutamic acid, glutaric acid, glucuronic acid, aspartic acid, ascorbic acid, carbonic acid, vanillic acid, hydroiodic acid, pyruvic acid, oxalic acid, malic acid, malonic acid, lactic acid, succinic acid, maleic acid, fumaric acid, tartaric acid, citric acid, benzoic acid, cinnamic acid, mandelic acid, methanesulfonic acid, ethanesulfonic acid, benzenesulfonic acid, p-toluenesulfonic acid, naphthalenesulfonic acid and/or salicylic acid, and the like.

The pharmaceutically acceptable salt may be a hydrochloride salt.

The pyrazole-based compound represented by Formula 1 or a pharmaceutically acceptable salt thereof included in the pharmaceutical composition of the present invention is specifically exemplified as follows:
3-phenyl-4-methyl-1-(pyridin-2-yl)-1*H*-pyrazol-5-ol or a hydrochloride salt thereof;
3-phenyl-4-ethyl-1-(pyridin-2-yl)-1*H*-pyrazol-5-ol or a hydrochloride salt thereof;
3-phenyl-4-n-propyl-1-(pyridin-2-yl)-1*H*-pyrazol-5-ol or a hydrochloride salt thereof;
3-phenyl-4-isopropyl-1-(pyridin-2-yl)-1*H*-pyrazol-5-ol or a hydrochloride salt thereof;
3-phenyl-4-n-butyl-1-(pyridin-2-yl)-1*H*-pyrazol-5-ol or a hydrochloride salt thereof;
3-phenyl-4-*tert*-butyl-1-(pyridin-2-yl)-1*H*-pyrazol-5-ol or a hydrochloride salt thereof;
3-phenyl-4-n-pentyl-1-(pyridin-2-yl)-1*H*-pyrazol-5-ol or a hydrochloride salt thereof;
3-phenyl-4-n-hexyl-1-(pyridin-2-yl)-1*H*-pyrazol-5-ol or a hydrochloride salt thereof.

Specifically, the pyrazole-based compound included in the pharmaceutical composition of the present invention may be 3-phenyl-4-n-propyl-1-(pyridin-2-yl)-1*H*-pyrazol-5-ol or a hydrochloride salt thereof.

The compound of Formula 1 of the present invention may inhibit the generation of reactive oxygen species in kidney tissue.

In the present invention, oxidative stress refers to tissue damage caused by a relatively excessive production of reactive oxygen species as the balance between the production of reactive oxygen species (ROS) and the antioxidant defense mechanism for biomolecules, cells and tissues is broken. In this case, "reactive oxygen species" may refer to activated oxygen, active oxygen, and activated oxygen species, which refer to the same substance.

The "contrast media" of the present invention are known to those skilled in the art. Contrast media allow a specific part of the body to be distinguished from a periphery of similar density. Preferably, the contrast media in the context of the present invention is an opaque or positive contrast media, i.e., a contrast media which has a greater attenuation density than the surrounding tissue, thus enhancing the absorption of x-rays.

Positive contrast media is well known in the art and are non-iodine-based contrast media, iodine-based contrast media, i.e., iodized contrast media. Examples of non-iodine-based and iodine-based contrast media are known to those skilled in the art.

Monomeric contrast media is divided into ionic and non-ionic, and specific ionic monomeric contrast media include ioglycate (Rayvist), iodamide (Uromiro), acetrizoate (Diaginol, Urokon), diatrizoate (Angiografin; Hypaque; Renografin; Urografin; Urovison), and metrizoate (Isopaque; Triosil).

Specific non-ionic monomeric contrast media include metrizamide (Amipaque), iohexol (Omnipaque), iopamidol (Iopamiro), iopenthol (Imagopaque), iopromide (Ultravist), and ioversol (Optiray) .

Dimeric iodine-based contrast media preferably contains two tri-iodinated benzene rings. They may be grouped into ionic intravenous cholerographic contrast media, monoacidic ionic contrast media, and non-ionic contrast media. The dimeric iodinated contrast media is preferably ioxaglic acid (Hexabrix), iotrolan (Isovist), and iodixanol (Visipaque; Optiprep).

Non-iodine contrast media often contains barium, primarily in the form of insoluble barium sulfate. They are preferably administered for gastrointestinal tract examination.

Contrast media may be administered by any method deemed appropriate. Those skilled in the art are aware that the method selected for administration may depend on the purpose of the examination for which the contrast media is administered, and/or the contrast media. Specifically, the barium-based contrast media is administered by swallowing or in the form of an enema. The iodine-based contrast media is preferably administered by injection into a vein, spinal canal or artery. A catheter for administration of iodine-based contrast media may be used.

Administration of iodine-based contrast media is for computed tomography, and most preferably angiography. Administration of contrast media suitable for ultrasonography or magnetic resonance imaging (MRI) is further considered in the present invention.

"Acute kidney injury" or "AKI" or "acute renal failure (ARF)" of the present invention are well known in the art. As used in the present invention, the terms refer to the rapid loss of renal function. The rapid loss of renal function is caused by injury to the kidney(s). Criteria for diagnosis and classification of AKI are based on changes in serum creatinine levels and urinary excretion. The terms are defined in the KDIGO Guidelines (KDIGO, Kidney International Supplements(2012) 2, 69-88), which is incorporated herein by reference in its entirety.

Contrast media-induced acute kidney injury is defined as an increase in the level of creatinine in the blood by 25% or more or 0.5 mg/dl or more compared to the existing level within 48 hours of using contrast media, not a decrease in renal function due to other causes, such as hypotension, use of other nephrotoxic drugs, urinary tract obstruction, and embolism.

In general, contrast media may exhibit side effects such as decreased renal function in some cases even when administered to a person with normal renal function, but may more easily show deterioration of renal function and aggravate reduced renal function, especially when administered to persons with decreased renal function.

Although the exact mechanism of contrast media-induced acute kidney injury has not been fully elucidated, it is known that contrast media increases osmotic pressure, decreases renal blood flow, and causes renal artery constriction. In addition, renal tubular necrosis or decreased renal perfusion is known as the cause of contrast media-induced acute kidney injury. It is known that in this state, the generation of reactive oxygen species is promoted and ischemic tubular injury is caused, which may be the direct cause of tubular toxicity. In addition, inflammation is known as one of the causes.

Therefore, in order to prevent or treat contrast media-induced acute kidney injury, although the renal protective effect by the sufficient fluid supply and the treatment with antioxidants such as bicarbonate, N-acetylcysteine or ascorbic acid to reduce reactive oxygen species has been reported, there is no established treatment method yet, and prevention is the only treatment method.

The pharmaceutical composition of the present invention, in particular, all of 3-phenyl-4-ethyl-1-(pyridin-2-yl)-1H-pyrazol-5-ol, 3-phenyl-4-n-propyl-1-(pyridin-2-yl)-1*H*-pyrazol-5-ol and 3-phenyl-4-n-butyl-1-(pyridin-2-yl)-1*H*-pyrazol-5-ol showed an inhibitory effect on the formation of reactive oxygen species.

As confirmed by animal experiments in the examples of the present invention, the serum creatinine concentration, which is an indicator of contrast media-induced acute kidney injury, was significantly reduced, and the degree of renal tubular injury was also reduced. In addition, neutrophil gelatinase-associated lipocalin (NGAL), kidney injury molecule-1 (KIM-1) and microproteinuria, which are markers of renal injury, were reduced, and infiltration of inflammatory cells within kidney tissue, and activated oxygen and nitrotyrosine, which are indicators of oxidative stress, were reduced.

Therefore, in the contrast media-induced acute kidney injury model, the effect of preventing or alleviating acute kidney injury by suppressing the generation of active oxygen in the kidney tissue to inhibit the inflammatory response was confirmed.

The pharmaceutical composition of the present invention may further comprise a bicarbonate and/or an antioxidant for removing reactive oxygen species, in addition to the compound of Formula 1 or a salt thereof.

The pharmaceutical composition of the present invention may comprise a pharmaceutically acceptable carrier within a range that does not impair the effects of the present invention.

The "pharmaceutically acceptable carrier" includes any and all kinds of solvents, dispersion media, coatings, surfactants, antioxidants, preservatives (antibacterial or antifungal agents), isotonic agents, diluents, absorption delaying agents, salts, preservatives, stabilizers, binders, excipients, disintegrants, lubricants, sweetening agents, flavouring agents, dyes, and the like, and combinations thereof, as known to those skilled in the art.

The diluent may be selected from the group consisting of, but is not limited to, microcrystalline cellulose, lactose monohydrate, lactose anhydride, lactose, starch, mannitol, carboxymethylcellulose, sorbitol, and combinations thereof.

The disintegrant may be selected from the group consisting of, but is not limited to, low-substituted hydroxypropyl cellulose, crospovidone, croscarmellose sodium, sodium starch glycolate, F-melt, and combinations thereof.

The binder may be selected from the group consisting of, but is not limited to, hydroxypropyl cellulose, hydroxypropyl methylcellulose, hypromellose, polyvinyl acetic acid, povidone, polyvinylpyrrolidone, copovidone, macrogol, sodium lauryl sulfate, light anhydrous silicic acid, synthetic aluminum silicate, silicate derivatives such as calcium silicate or magnesium metasilicate aluminate, phosphates such as calcium hydrogen phosphate, carbonates such as calcium carbonate, pregelatinized starches, gums such as acacia gum, gelatin, cellulose derivatives such as ethyl cellulose, and mixtures thereof.

The lubricant may be selected from the group consisting of, but is not limited to, magnesium stearate, silicon dioxide, talc, light anhydrous silicic acid, sodium stearyl fumarate, and combinations thereof.

As a pH adjusting agent, an acidifying agent such as acetic acid, adipic acid, ascorbic acid, sodium ascorbate, sodium etherate, malic acid, succinic acid, tartaric acid, fumaric acid and citric acid, and a basifying agent such as aqueous ammonia, sodium carbonate, magnesium oxide, magnesium carbonate, sodium citrate and tribasic calcium phosphate may be used.

As the antioxidant, dibutyl hydroxy toluene, butylated hydroxyanisole, tocopherol acetate, tocopherol, propyl gallate, sodium hydrogen sulfite, sodium pyrosulfite and the like may be used.

In addition, it is possible to formulate the agents of the present invention by selectively using various additives selected from colorants and flavourings as pharmaceutically acceptable additives.

In the present invention, the scope of the additives is not limited to using the additives, and it may be formulated to contain a dose within a normal range by selectively using the additives.

The pharmaceutical composition according to the present invention may be formulated and used in the form of oral formulations such as powders, granules, tablets, capsules, suspensions, emulsions, syrups and aerosols, external preparations, suppositories, or sterile injectable solutions by a conventional method.

In one aspect of the present invention, it is a pharmaceutical composition for preventing, improving or treating contrast media-induced acute kidney injury, comprising the active ingredient in the range of 0.00001 to 100% by weight, 0.0001 to 95% by weight, or 0.001 to 90% by weight based on the total weight of the pharmaceutical composition.

In the preventive or therapeutic agent for contrast media-induced acute kidney injury according to the present invention, the dosage of the pyrazole-based compound represented by Formula 1 or a pharmaceutically acceptable salt thereof may be appropriately changed depending on the age and body weight of the patient, the symptoms, the route of administration, and the like.

The dosage of the pyrazole-based compound represented by Formula 1 or a pharmaceutically acceptable salt thereof of the present invention may be 0.00001 mg/kg/day to 2000 mg/kg/day, 0.0001 mg/kg/day to 1000 mg/kg/day, 0.001 mg/kg/day to 800 mg/kg/day, 0.001 mg/kg/day to 500 mg/kg/day, 0.001 mg/kg/day to 100 mg/kg/day, 0.001 mg/kg/day to 80 mg/kg/day, or 0.01 mg/kg/day to 70 mg/kg/day.

The content of the pyrazole-based compound represented by Formula 1 or a pharmaceutically acceptable salt thereof of the present invention may be 0.00001 to 100% by weight, 0.0001 to 95% by weight, 0.0001 to 90% by weight, 0.001 to 70% by weight, or 0.001 to 50% by weight per unit dosage form.

The administration concentration of the pyrazole-based compound represented by Formula 1 or a pharmaceutically acceptable salt thereof of the present invention may be 0.0001 to 500 µM, 0.001 to 300 µM, 0.001 to 150 µM, 0.001 to 130 µM, 0.001 to 100 µM, 0.001 to 80 µM, or 0.01 to 70 µM.

The pharmaceutical composition of the present invention may be administered together with or separately from contrast media through a general route, and may be specifically formulated for intramuscular, intrathecal, intra-digestive, intracardiovascular, intrarenal, or intravenous administration. Formulation methods employ conventional methods known to those skilled in the art.

A conventional composition for intramuscular or intrathecal administration may consist of, but is not limited to, for example, the active ingredient and a sterile isotonic aqueous solution containing dextrose, sodium chloride, or both dextrose and sodium chloride. Other examples include, but are not limited to, lactated Ringer's injection, lactated Ringer's injection + dextrose injection, Normosol-M and dextrose, Isolyte E, acylated Ringer's injection, and the like. Optionally, the present formulation may comprise, but is not limited to, a cosolvent such as polyethylene glycol; chelating agents such as ethylenediamine tetraacetic acid; and antioxidants such as sodium metabisulphite. Optionally, without limitation, the solution may be lyophilized and then reconstituted with a suitable solvent immediately prior to administration.

Preferred examples are provided to help understanding of the present invention. The following examples are provided not to limit the present invention but to facilitate the understanding of the present invention.

### [Mode for Carrying out the Invention]

### <Synthetic Examples>

### <Synthesis Example 1> Synthesis of 3-phenyl-4-ethyl-1-(pyridin-2-yl)-1H-pyrazol-5-ol

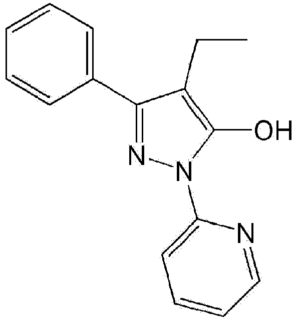

In a round bottom flask, 2-ethyl-3-oxo-3-phenylpropionic acid ethyl ester (10.7 g, 49 mmol) and 2-hydrazinopyridine (5.6 g, 51.4 mmol) were heated to reflux under nitrogen condition without a solvent for 1 day. The resulting solid was purified with hexane and ethyl acetate and then dried under vacuum to obtain the title compound in a yield of 70%.

1H NMR(300 MHz, DMSO-d6) δ 8.25-8.24(1H, d), 8.00-7.97(1H, d), 7.84-7.82(1H, t), 7.73-7.71(2H, m), 7.46-7.37(3H, m) 7.12-7.11(1H, t), 2.62-2.57(2H, m), 1.23-1.17(3H, m); ESI(m/z) 266.1[M+H]⁺

### <Synthesis Example 2> Synthesis of 3-phenyl-4-butyl-1-(pyridin-2-yl)-1H-pyrazol-5-ol

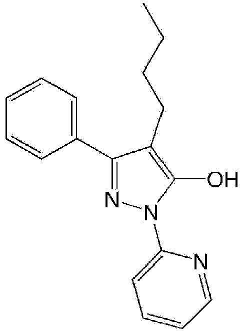

In a round bottom flask, 2-butyl-3-oxo-3-phenylpropionic acid ethyl ester (12.1 g, 49 mmol) and 2-hydrazinopyridine (5.6 g, 51.4 mmol) were heated to reflux under nitrogen condition without a solvent for 1 day. The resulting solid was purified with hexane and ethyl acetate and then dried under vacuum to obtain the title compound in a yield of 75%.

1H NMR(300 MHz, DMSO-d6) δ 8.25-8.24(1H, d), 8.03-8.02(1H, d), 7.85-7.83(1H, t), 7.70-7.69(2H, m), 7.44-7.35(3H, m) 7.12-7.11(1H, t), 2.56-2.53(2H, t), 1.58-1.52(2H, m), 1.38-1.24(2H, m), 0.89-0.86(3H, t); ESI(m/z) 294.0[M+H]⁺
[

### <Synthesis Example 3> Synthesis of 3-phenyl-4-propyl-1-(pyridin-2-yl)-1H-pyrazol-5-ol

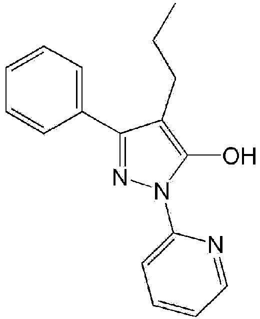

2-Propyl-3-oxo-3-phenylpropionic acid ethyl ester (2.52 g, 10.7 mmol) and 10 ml of ethanol were placed in a round bottom flask, and then a solution of 2-hydrazinopyridine (1.29 g, 1.18 mmol) diluted in 3 ml of ethanol was slowly added dropwise thereto at 0°C. It was heated to reflux at 100°C for 3 days. The solvent was removed by distillation under reduced pressure, and the resulting solid was washed with hexane and ethyl acetate, and then dried under vacuum to obtain the title compound in a yield of 82%.

1H NMR(300 MHz, CDCl₃) δ 12.50(1H, s), 8.27-8.25(1H, m), 8.01(1H, d, J = 8.5 Hz), 7.81(1H, m), 7.69(2H, m), 7.48-7.34(3H, m), 7.12-7.10(1H, m), 2.54(2H, d, J= 7.5 Hz), 1.64(2H, m), 0.93(3H, t, *J* = 7.3 Hz); EIMS(70 eV) m/z(rel intensity) 279(M+, 37), 250(100)

### <Synthesis Example 4> Synthesis of 3-phenyl-4-propyl-1-(pyridin-2-yl)-1H-pyrazol-5-ol hydrochloride (Compound 1)

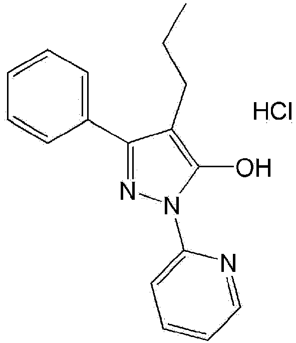

3-Phenyl-4-propyl-1-(pyridin-2-yl)-1*H*-pyrazol-5-ol (280 mg, 1.0 mmol) prepared in Synthesis Example 3 above was dissolved in 4 ml of ethyl ether in a round bottom flask, and then 0.55 ml of ethyl ether dissolved in 2 M HCl was slowly added dropwise thereto at 0°C. The solid produced from the reaction solution was filtered under reduced pressure, the solvent was removed, washed with hexane and ethyl acetate, and then dried under vacuum to obtain the title compound (270 mg, 0.85 mmol).

1H NMR(300 MHz, CDCl₃) δ 8.44(1H, d, *J* = 4.2 Hz), 8.0-8.03 (2H, m), 7.66-7.64(2H, m), 7.48-7.42(3H, m), 7.34-7.30(1H, m), 2.49(2H, brs), 2.43 (2H, t, J = 7.5 Hz), 1.48(2H, m), 0.48(3H, t, J = 7.3 Hz)

### <Example> Efficacy evaluation of animal model with contrast media-induced acute kidney injury in mice

As the experimental animals, 6-week-old male C57BL/6 mice were purchased from LionBio. All mice were maintained under standard conditions. After breeding for about 2 weeks, at 8 weeks of age, Compound 1 (3-phenyl-4-n-propyl-1-(pyridin-2-yl)-1H-pyrazol-5-ol hydrochloride) was orally administered at a dose of 60 mg/kg daily for 5 days before administration of contrast media.

In order to induce acute kidney injury, after a fast of 16 hours, the anti-inflammatory analgesic indomethacin (10 mg/kg) and the NO blocker N-nitro-L-arginine (L-NAME, 10 mg/kg) were intraperitoneally administered 15 minutes before administration of contrast media, and contrast media (Iohexol, 4000 mg iodine/kg) was intraperitoneally administered 15 minutes later. After 24 hours of supplying food and water, the animal test substances were classified into three groups: vehicle administered group (Control) and contrast media administered group (Iohexol), and contrast media and Compound 1 simultaneously administered group (Iohexol + Compound 1).

After completion of the test, the animals were anesthetized, kidney tissue was enucleated for each individual, and blood was taken for examination. The tissues were fixed in 10% buffered neutral formalin solution. The fixed tissues were sliced to a certain thickness and paraffin-embedded through a general tissue processing process to produce a tissue section of 4 to 5 µm, and then hematoxylin & eosin staining (H&E stain), which is a general staining method, was performed to observe histopathologically the extent of kidney injury in the region of the renal cortex and renal medulla, and F4/80 staining, Tunel staining, DHE staining and nitrotyrosine staining were performed to observe the effects of inflammation and oxidative stress in the kidney injury tissue.

Blood was taken and centrifuged at 3000 rpm, 4°C for 10 minutes, and the upper layer of serum was taken and the level of blood urea nitrogen (BUN) and creatinine was measured using an automatic blood biochemical analyzer (AU480, Beckman Coulter, USA) to confirm the indicator of kidney injury. Histologic evaluation of the degree of kidney injury was calculated as 0 point for no renal tubular injury, 1 point for mild injury, 2 points for intratubular vacuolar lesions less than 25-60%, and 3 points for intratubular vacuolar lesions of 60% or more, thereby formulating the average value of renal tubular injury under the microscope field of view.

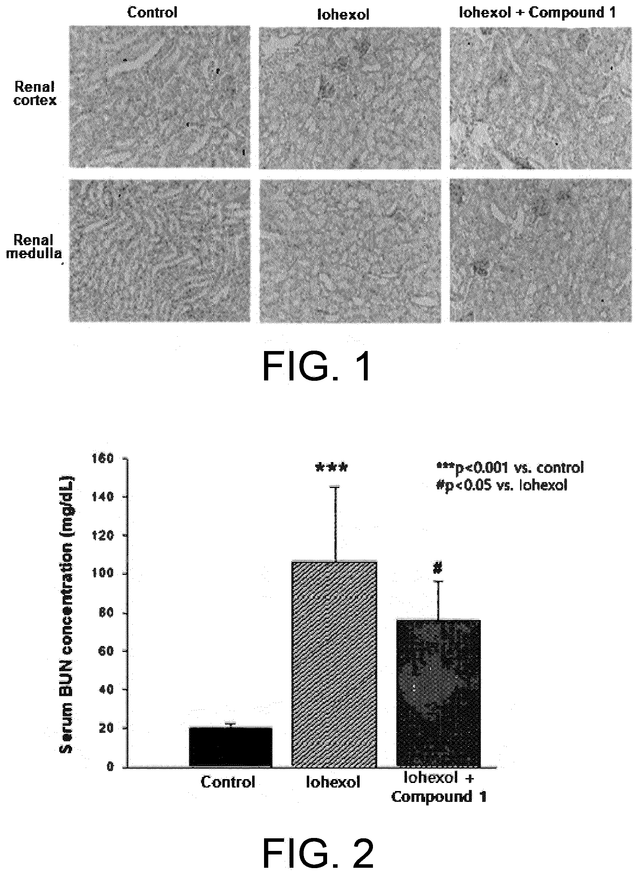

Figure 1 shows photographs showing the effect of reducing renal tubular injury in kidney tissue in the group administered with Compound 1 at 60 mg/kg once a day for 5 days in an evaluation using animal model with contrast media-induced acute kidney injury induced in mice. As shown in Figure 1, as a result of collecting kidney tissue and confirming the extent of kidney injury in the region of the renal cortex and renal medulla, it was found that there were vacuolar change due to injury of tubule cells and severe renal tubular tissue injury due to the loss of brush border in the contrast media-treated group compared to the normal control group, and the kidney injury of the renal medulla and the renal cortex was remarkably reduced in the group treated with Compound 1.

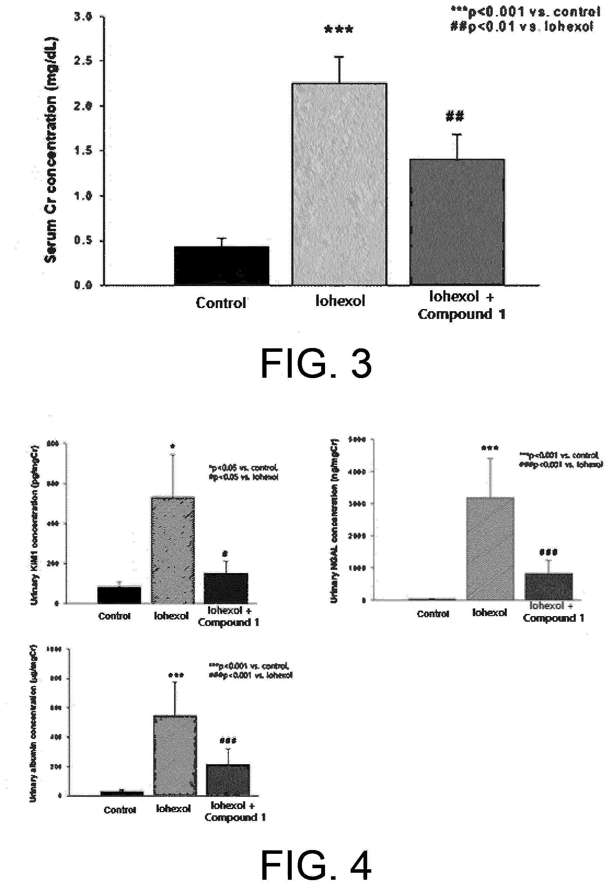

Figures 2 and 3 show graphs showing the effect of reducing kidney injury in blood test in the group administered with Compound 1 at 60 mg/kg once a day for 5 days in an evaluation using animal model with contrast media-induced acute kidney injury induced in mice. As shown in Figures 2 and 3, it was found that the concentration of BUN and creatinine (Serum Cr) was remarkably increased in the group treated with the contrast media compared to the normal control group, and the concentration of BUN and creatinine was decreased in the group treated with Compound 1, in particular, the creatinine concentration was decreased, which reduced the kidney injury.

Figure 4 shows graphs showing the effect of reducing the kidney injury markers NGAL, KIM-1 and microproteinuria (albumin) in the urine in the group administered with Compound 1 at 60 mg/kg once a day for 5 days in an evaluation using animal model with contrast media-induced acute kidney injury induced in mice. As shown in Figure 4, it was found that the concentration of NGAL, KIM-1 and microproteinuria (albumin) was remarkably increased in the group treated with the contrast media compared to the normal control group, and the concentration of NGAL, KIM-1 and microproteinuria (albumin) was decreased in the group treated with Compound 1, which reduced the kidney injury.

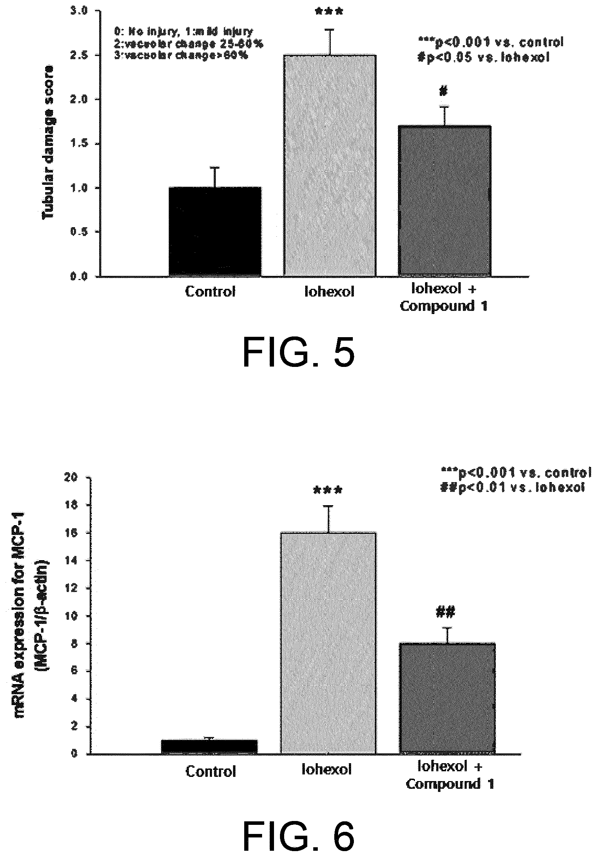

Figure 5 shows a graph showing the effect of reducing renal tubular injury in a renal biopsy in the group administered with Compound 1 at 60 mg/kg once a day for 5 days in an evaluation using animal model with contrast media-induced acute kidney injury induced in mice. Figure 5 shows a result of collecting kidney tissue and comparing the degree of renal tubular injury reduction, and it was found that the renal tubular injury score was remarkably increased in the contrast media-treated group compared to the normal control group, and the renal tubular injury score was remarkably reduced in the group treated with Compound 1.

Figure 6 shows a graph showing the effect of reducing inflammation in the kidney tissue in the group administered with Compound 1 at 60 mg/kg once a day for 5 days in an evaluation using animal model with contrast media-induced acute kidney injury induced in mice. Figure 6 shows the analysis of the expression level of monocyte chemoattractant protein-1 (MCP-1) gene, which is an indicator of inflammation in the kidney tissue, and it was found that the expression of MCP-1 was increased in the group treated with contrast media compared to the normal control group, whereas inflammation (MCP-1) is remarkably reduced in the group treated with Compound 1.

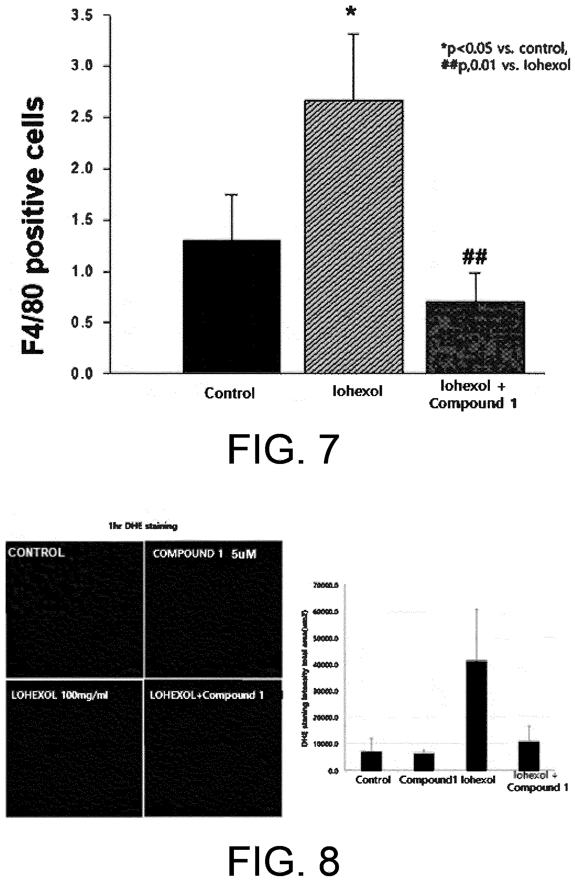

Figure 7 shows the analysis of the degree of infiltration of inflammatory cells (macrophages, F4/80-positive cells) within the kidney tissue in the group administered with Compound 1 at 60 mg/kg once a day for 5 days in an evaluation using animal model with contrast media-induced acute kidney injury induced in mice, it was found that the infiltration of inflammatory cells was remarkably increased in the group treated with contrast media compared to the normal control group, and the infiltration of inflammatory cells was remarkably reduced in the group treated with Compound 1 compared to the group treated with contrast media.

Figure 8 shows the analysis of the degree of reactive oxygen species (DHE stainig), which is an indicator of oxidative stress in the kidney tissue, in the group administered with Compound 1 at 60 mg/kg once a day for 5 days in an evaluation using animal model with contrast media-induced acute kidney injury induced in mice, it was found that activated oxygen was remarkably increased in the group treated with contrast media compared to the normal control group, and activated oxygen was remarkably reduced in the group treated with Compound 1 compared to the group treated with contrast media.

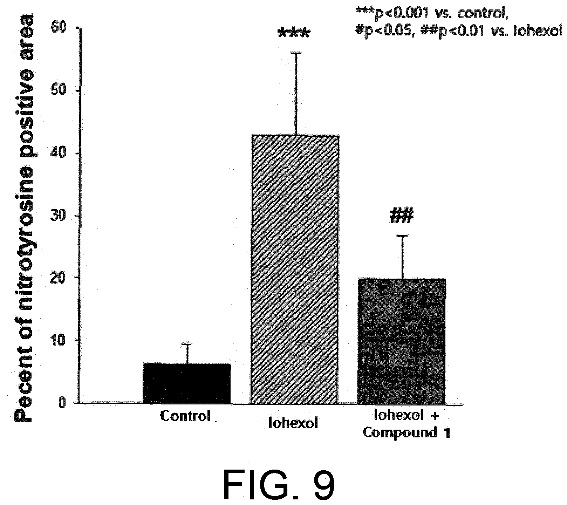

Figure 9 shows the analysis of the degree of nitrotyrosine, which is an indicator of oxidative stress in the kidney tissue, in the group administered with Compound 1 at 60 mg/kg once a day for 5 days in an evaluation using animal model with contrast media-induced acute kidney injury induced in mice, it was found that nitrotyrosine was remarkably increased in the group treated with contrast media compared to the normal control group, and nitrotyrosine was remarkably reduced in the group treated with Compound 1 compared to the group treated with contrast media.

Therefore, it was confirmed that the compound of this application has a remarkable renal protective effect in the acute kidney injury model caused by contrast media.

## Claims

1. A pharmaceutical composition for protecting a kidney by reducing contrast media-induced nephrotoxicity or for preventing or treating contrast media-induced acute kidney injury, comprising a compound of following Formula 1 or a pharmaceutically acceptable salt thereof: 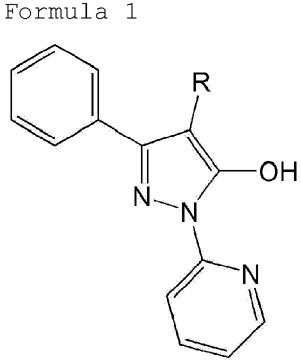 wherein R is a linear or branched alkyl group having 1 to 10 carbon atoms.

2. The pharmaceutical composition according to claim 1, wherein in Formula 1, R is a linear or branched alkyl group having 1 to 6 carbon atoms.

3. The pharmaceutical composition according to claim 2, wherein the compound of Formula 1 is:
3-phenyl-4-methyl-1-(pyridin-2-yl)-1*H*-pyrazol-5-ol or a hydrochloride salt thereof;
3-phenyl-4-ethyl-1-(pyridin-2-yl)-1H-pyrazol-5-ol or a hydrochloride salt thereof;
3-phenyl-4-n-propyl-1-(pyridin-2-yl)-1*H*-pyrazol-5-ol or a hydrochloride salt thereof;
3-phenyl-4-isopropyl-1-(pyridin-2-yl)-1*H*-pyrazol-5-ol or a hydrochloride salt thereof;
3-phenyl-4-n-butyl-1-(pyridin-2-yl)-1*H*-pyrazol-5-ol or a hydrochloride salt thereof;
3-phenyl-4-*tert*-butyl-1-(pyridin-2-yl)-1*H*-pyrazol-5-ol or a hydrochloride salt thereof;
3-phenyl-4-n-pentyl-1-(pyridin-2-yl)-1*H*-pyrazol-5-ol or a hydrochloride salt thereof; or
3-phenyl-4-n-hexyl-1-(pyridin-2-yl)-1*H*-pyrazol-5-ol or a hydrochloride salt thereof.

4. The pharmaceutical composition according to claim 3, wherein the compound of Formula 1 is 3-phenyl-4-n-propyl-1-(pyridin-2-yl)-1*H*-pyrazol-5-ol or a hydrochloride salt thereof.

5. The pharmaceutical composition according to any one of claims 1 to 4, wherein the contrast media is selected from an ionic monomeric contrast media or a non-ionic monomeric contrast media.

6. The pharmaceutical composition according to claim 5, wherein the ionic monomeric contrast media is selected from ioglycate, iodamide, acetrizoate, diatrizoate, and metrizoate.

7. The pharmaceutical composition according to claim 5, wherein the non-ionic monomeric contrast media is selected from metrizamide, iohexol, iopamidol, iopenthol, iopromide, and ioversol.

8. The pharmaceutical composition according to claim 7, wherein the non-ionic monomeric contrast media is iohexol.

9. The pharmaceutical composition according to any one of claims 1 to 4, wherein the acute kidney injury is caused by renal tubular necrosis or decreased renal perfusion.

10. The pharmaceutical composition according to any one of claims 1 to 4, wherein the acute kidney injury is caused by oxidative stress or inflammation.

11. The pharmaceutical composition according to any one of claims 1 to 4, wherein the pharmaceutical composition further comprises a pharmaceutically acceptable carrier or excipient.
